# EUROPÄISCHE PATENTANMELDUNG

(11) **EP 0 682 979 A1**
(43) Veröffentlichungstag der Anmeldung: **22.11.1995**
(21) Anmeldenummer: 95104103.7
(22) Anmeldetag: 20.03.1995
(51) Int. Cl.: B01F 3/04

(54) **Vorrichtung zur Einspeisung von Kohlendioxid in Leitungswasser**

(30) Priorität: 21.03.1994 DE 9404731 U
(71) Anmelder: Negele, Helfried, D-86916 Kaufering (DE)
(72) Erfinder: Negele, Helfried, D-86916 Kaufering (DE)
(74) Vertreter: Kirschner, Klaus Dieter, Dipl.-Phys.

(57) **Zusammenfassung**

Die Vorrichtung zur Einspeisung von CO₂ in Leitungswasser hat eine CO₂ -Quelle (1), die gasförmiges Kohlendioxid unter Druck bereitstellt und aus der das CO₂-Gas dem über eine Wasserleitung (8) zugeführten Leitungswasser vor einer Wasserabgabestelle zugemischt wird. Die CO₂-Quelle ist eine handelsübliche CO₂-Gasflasche, und die CO₂-Gasflasche ist über eine Druckleitung (5) mit einem Anschluß eines T-Stücks (6) verbunden, dessen andere Anschlüsse in der Wasserleitung liegen.

## Beschreibung

Die Erfindung bezieht sich auf ein Vorrichtung zur Einspeisung von Kohlendioxid in Leitungswasser nach dem Oberbegriff des Hauptanspruches.

Es ist bekannt, daß die Haut und das Haar durch den ständigen Einfluß von umweltbelastenden Faktoren ebenso wie die zum Waschen oder Tönen bzw. Färben verwendeten Mittel stark beansprucht wird, wodurch deren natürliche Struktur und insbesondere das Wachstum der Haare stark beeinträchtigt wird.

Leitungswasser ist zur Pflege der Haut und der Haare nur bedingt geeignet, da es einen pH-Wert zwischen 7 und 8,2 aufweist. Da das Haar und die Haut einen pH-Wert von 5,7 haben, wirkt das Leitungswasser quellend auf deren Oberfläche. Eine Quellung von Haut und Haar ist nicht zu vermeiden, so daß Bakterien, Pilze, Viren und Umwelteinflüsse direkt in die gequollenen Hautporen eindringen können.

Um diesen Nachteil zu beseitigen, ist es bekannt, Kohlendioxid in Leitungswasser einzuspeisen. Die dabei durch Lösung von Kohlendioxid in Wasser entstehende Kohlensäure senkt den pH-Wert des Leitungswassers auf Werte zwischen 5,5 und 6,0 ab. Zur Einspeisung von Kohlendioxid in Leitungswasser offenbart die DE-PS 41 24 728 ein Verfahren zur Haarpflege, bei dem das zufließende Wasser vor Erreichen der Wasserentnahmestelle vorübergehend stark beschleunigt, CO₂-Gas in Abhängigkeit von dem zufließenden Wasser einer Druckgaspatrone entnommen und auf das beschleunigt fließende Wasser direkt zur Einwirkung gebracht wird, so daß sich das Verhältnis zwischen Wasser- und Gasdruck auf einen bestimmten Wert einstellt.

Dieses Verfahren ist jedoch technisch sehr aufwendig, insbesondere wegen der erforderlichen Mischbatterie, und erfordert eine teuere und wartungs- sowie steuerungsbedürftige Anlage.

Weiterhin steht dieses Verfahren in der langen Tradition, daß zur Einspeisung von Gas in Flüssigkeit eine besondere Einspeisvorrichtung nötig sei, die am Einspeispunkt ganz bestimmte Strömungs- und Druckverhältnisse sicherstellt und zu diesem Zweck mit Kammern, Verengungen, Düsen oder dgl. versehen ist.

So wird in der DE-PS-41 24 728 großen Wert darauf gelegt, daß Schwankungen in der Strömungsgeschwindigkeit oder dem Druck des Wassers aus der Versorgungsleitung berücksichtigt werden. Dazu sind besondere Wasser-Vorkammern und genau abgemessene Strömumgskanäle vorgesehen, die die Strömungsgeschwindigkeit an bestimmten Stellen vergrößern und den statischen Druck senken sollen.

Ähnlich kompliziert ist die Vorrichtung in DE 34 32 440 C2, die zur Aufbereitung von Gießwasser mit CO₂ vorgesehen ist. Die dort beschriebene Vorrichtung soll gewährleisten, daß der Gasdruck, der für die Fließgeschwindigkeit und den Leitungsdruck im Gießwasser-Abgabesystem maßgeblich ist, erreicht wird, und daß der jeweilige partielle Druck in den Injektordüsen einen gewissen vorgegebenen Wert nicht überschreitet.

Dazu umfaßt die Vorrichtung ein System von mehreren hintereinander geschalteten Injektionsdüsen, die jeweils schrittweise und abrupt sich ändernde Strömungsquerschnitte aufweisen. Gemäß der offenbarten Lehre ist dies notwendig, um eine Mischung von Gas und Wasser zu erreichen.

Diese stufenförmige Erweiterung des Durchström-Querschnittes findet sich in einer ähnlichen Vorrichtung aus DE 34 10 621.

Eine weitere Anordnung zum Imprägnieren von Gießwasser mit CO₂ ist aus DE 33 04 403 bekannt, wobei hier jedoch mehrere Stellen zum Einleiten von Kohlendioxyd in das Wasser vorgesehen sind, die über die Länge der Imprägnierzone verteilt sind und das Kohlendioxyd mit einer vorbestimmten Impulsfrequenz impulsförmig zuleiten. Weiterhin ist dort eine Strömungsdrosselung vorgesehen.

In einem Verfahren zur Wurzeldüngung von Kulturpflanzen offenbart DE 37 20 621 gilt die Einführung des Wassers in eine Immprägnierzone mit im wesentlichen turbulenzfreier Strömung als wesentliches Element, wobei durch schulterartige Querschnittserweiterungsstellen der Druck momentan herabgesetzt werden soll.

In DE 33 22 253 ist eine weitere Anordnung zum Imprägnieren von Gießwasser mit Kohlendioxyd beschrieben. Auch hier wird eine komplizierte Imprägniervorrichtung für notwendig gehalten, die einen Druckentspannungskegel in Kombination mit einem Druckminderventil umfaßt.

In die gleiche Richtung weist auch DE 33 30 375, das ein Verfahren und eine Anordnung zum Imprägnieren einer Flüssigkeit mit einem Gas durch Injektor-Wirkung offenbart, wobei in der Imprägnierungszone ein Injektordüsen-System vorgesehen ist, das die Flüssigkeit durch mehrere hintereinander geschaltete Injektorstufen mit stufenartig abnehmender Strömungsgeschwindigkeit leitet, wobei die plötzliche Änderung der Durchflußgeschindigkeit von Stufe zu Stufe eine innige Durchmischung von Gas und Flüssigkeit bewirken soll.

Eine Aufbereitungsanlage für Wasser mittels Einleitung von Kohlendioxyd ist in DE 34 36 660 offenbart, wobei zwei verschiedene Entnahme- und Verbraucherstellen, die unterschiedlich stark CO₂-imprägniertes Wasser abgeben, vorgesehen sind. Auch hier ist in der Imprägnierzone das bekannte abgestufte Injektordüsen-System eingesetzt, das sich auch in DE 37 30 552 und EP 0 155 683 wiederfindet.

Dagegen wird in DE 42 00 467 eine Beschleunigung des Wassers vor der Imprägnierung mit Kohlendioxyd in einem Verfahren und der Anordnung zur Haarpflege oder zur therapeutischen Behandlung der Haut für wesentlich gehalten.

Eine weitere Vorrichtung zum Mischen von flüssigen und/oder gasförmigen Stoffen oder Verbindungen ist aus DE 32 15 565 bekannt. Die Flüssigkeit und das Gas werden in einem Container gemischt, in welchem die Flüssigkeit steht.

Eine Vorrichtung zum Beimischen von flüssigen und/oder gasförmigen Zusätzen in Wasser ist in der deutschen Gebrauchsmusterschrift G 81 37 157 offenbart. Der Behälter mit dem Zusatzstoff ist an die Wasserleitung durch ein T-förmiges Rohrabzweigeelement angeschlossen, wobei das Rohrabzweigeelement aus zwei ineinander geschobenen Rohrteilen besteht. Das innere Rohrleitungsstück hat einen relativ geringen Durchmesser und der Ansatzteil für den Zusatzbehälter weist eine konische Bohrung auf. Diese Verengungen erfordern ein speziell gefertigtes Rohrabzweigelement.

In GB 1 604 326 ist ein Mischersystem zum Einmischen von Lösungen, Flüssigkeiten oder Gas in eine Flüssigkeit vorgesehen, wobei der Mischungspunkt durch eine konisch-geformte Düse gebildet wird, in welcher die einzuleitende Lösung beschleunigt wird.

Alle bekannten Vorrichtungen und Verfahren aus dem Stand der Technik gehen mithin davon aus, daß es für die erfolgreiche Einleitung von Gas in Flüssigkeit entscheidend auf die Druck- und Strömungsverhältnisse am Einspeispunkt ankommt und daher besondere Vorkehrungen, wie beispielsweise ein abgestuftes Injektionsdüsensystem erforderlich sind. Folglich hat sich die Entwicklung bisher darauf konzentriert, besonders geformte und genau dimensionierte Bauteile zum Einleiten des Gases in das Wasser zu konstruieren. Diese Bauteile sind nicht nur teuer in der Herstellung, sondern auch aufwendig in der Wartung und anfällig für Reparaturen.

Insbesondere bei der Einleitung von CO₂-Gas in Wasser zur Absenkung des PH-Wertes werden aufwendige Mechanismen verwendet, die einen gleichbleibenden PH-Wert des ausgegebenen Wassers sicherstellen sollen. Tatsächlich löst sich aber von dem CO₂-Gas ohnehin nur ein winziger Bruchteil in dem Wasser und dieser winzige Bruchteil kann auch mit sehr komplizierte Vorrichtungen nicht genau festgelegt werden, da er von vielen Faktoren u.a. der Qualität des Wassers abhängt.

Zudem gehen die bekannten Vorrichtungen von der festgefahrenen Vorstellung aus, daß die Eigenschaften des ausgegebenen Wassers an dem Einmischpunkt festgelegt werden können und müssen. Bei der Einleitung von CO₂-Gas findet jedoch die Lösung von CO₂ in Wasser über die ganze Wegstrecke statt, auf der das CO₂-Gas und das Wasser miteinander in Berührung stehen bis das Wasser am Ende ins Freie tritt und das übrige CO₂ verdunstet. Diese Strecke wird beispielsweise durch die Länge eines Schlauches zwischen Einspeisvorrichtung und Handgriff bei Verwendung in einer Dusche bestimmt. Kammern oder Düsen am Einspeispunkt haben dagegen keinen nennenswerten Einfluß.

Um den gewünschten Effekt zu erzielen, ist es daher von untergeordneter Bedeutung, wie die Druck- und Strömungsverhältnisse am Einleitpunkt beschaffen sind. Kurz gesagt, ist die bisherige technische Entwicklung in Bezug auf die Einspeisung von CO₂ in Leitungswasser in dem Irrtum befangen, daß durch eine besonders gestaltete Einspeisungsvorrichtung Wasser mit genau definierten Eigenschaften erhalten werden kann. Daher wurden immer neue und noch kompliziertere Einspeisvorrichtungen entwickelt, die zu dem erstrebten Ziel hinführen sollten.

Es ist daher die Aufgabe der Erfindung, eine einfache, kostengünstige und leicht zu installierende Anlage zur Einmischung von Kohlendioxyd in Wasser bereitzustellen.

Diese Aufgabe wird durch die Vorrichtung nach dem Hauptanspruch gelöst.

Nach einer speziellen Ausführungsform der Erfindung weist die Vorrichtung eine herkömmliche Druckgasflasche, die mit einem Druckminderer versehen ist, einen Druckschlauch zur Zuführung des Gases zu einem T-Stück, in dessen Leitung ein Ventil, vorzugsweise ein Kugelhahnventil, eingesetzt ist, sowie ein T-Stück auf, in das einerseits das Leitungswasser und andererseits das CO₂-Gas eingespeist werden, so daß sich die beiden Fluide im Auslaß des T-Stücks mischen, wodurch einer Wasserentnahmestelle, wie etwa einem Duschkopf, kohlensäurehaltiges Wasser zugeführt wird.

Die erfindungsgemäße Vorrichtung hat folglich den Vorteil, daß einerseits der pH-Wert des Leitungswassers auf Werte zwischen 5,5 und 6,0 abgesenkt wird, und daß andererseit aber handelsübliche Teile verwendet werden können, so daß die Vorrichtung preiswert zusammengestellt werden kann. Durch Verwendung einer handelsüblichen Druckgasflasche werden auch die Betriebskosten derart gesenkt, daß die Vorrichtung wirtschaftlich, beispielsweise in Friseursalons oder auch im Haushalt eingesetzt werden kann.

Eine vorteilhafte Ausgestaltung der erfindungsgemäßen Vorrichtung besteht darin, daß in der Druckleitung ein zusätzliches Absperrorgan, insbesondere ein Kugelhahnventil, vorgesehen ist. Dadurch kann die Druckgasflasche an einer Stelle entfernt von dem Ort, an dem das Wasser verbraucht wird, aufgestellt werden, was beispielsweise in einem Haushalt vorteilhaft ist.

Eine weitere vorteilhafte Ausgestaltung der erfindungsgemäßen Vorrichtung besteht darin, daß das Kugelhahnventil mit dem T-Stück integriert ist, wodurch das Ventil leicht zugänglich wird.

Weitere vorteilhafte Ausgestaltungen der Erfindung ergeben sich aus den restlichen Unteransprüchen

Nachfolgend wird ein bevorzugtes Ausführungsbeispiel der Erfindung unter Bezugnahme auf die beiliegenden Zeichnungen beschrieben. Es zeigen:
- Fig. 1: eine schematische Ansicht der Vorrichtung zur Einspeisung von Kohlendioxid in Wasser; und
- Fig. 2: einen schematischen Schnitt durch das T-Stück und das Ventil.

Die Vorrichtung besteht aus einer CO₂-Druckgasflasche 1, die mit einem Auslaßventil 2 sowie einem mit Manometern 3,3' ausgestatteten Druckminderer 4 versehen ist. Es gibt handelsübliche Druckgasflaschen, in denen das CO₂-Gas unter einem Druck von ca. 40 bis 50 bar steht und die ein Volumen von 2,7 1 beziehungsweise einen Inhalt von 2 kg CO₂-Gas haben, so daß ein genügend großer Vorrat an CO₂-Gas bei geringen Kosten zur Verfügung steht. Der Gasdruck wird in dem Druckminderer auf ca. 3,5 bis 5 bar Arbeitsdruck reduziert, sodaß ein ausreichender Arbeitsdruck zum Einmischen des CO₂-Gases in das Leitungswasser zur Verfügung steht.

Vom Druckminderer 4 führt ein Druckschlauch 5 zu einem T-Stück 6. Das T-Stück 6 ist mit einem Ventil 7, vorzugsweise einem Kugelhahnventil, verbunden, an das sich die Druckleitung 5 anschließt. Die anderen Anschlüsse des T-Stücks 6 liegen in der Wasserleitung, wobei beispielsweise an einen Anschluß ein Wasserschlauch angeschlossen werden kann, der beispielsweise zu einem Duschkopf oder einer anderen Wasserentnahmestelle führt. Das T-Stück 6 hat dazu an einem Anschluß ein Gewinde 8 zum Anschrauben an einen Wasserhahn, an einem weiteren Anschlup ein Gewinde 9 zum Anschrauben an einen Duschschlauch und an einem weiteren Anschluß eine Verbindung zum Anschluß an das Ventil 7. Der Schankel des T-Stückes 6, der an den Wasserhahn angeschlossen wird, steht unter einem spitzen Winkel zu dem Schenkel des T-Stückes 6, der am den Druckschlauch 5 angeschlossen ist, um das Einführen des Gases in das Wasser strömungsmäßig zu erleichern.

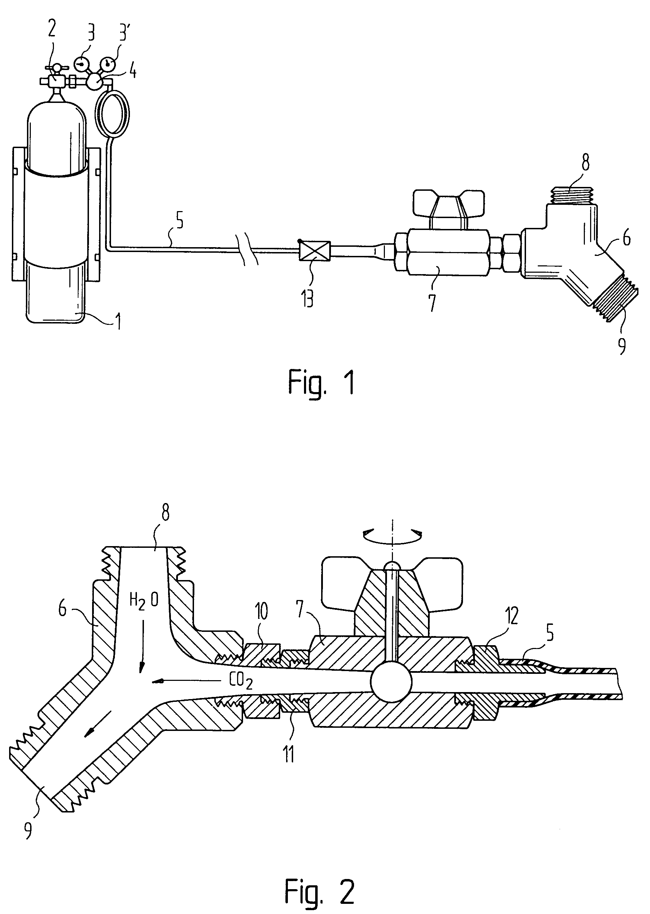

Das T-Stück und das Ventil sind zu einer Armatur zusammengefaßt, die gemäß Fig. 2 das T-Stück 6, ein erstes Reduzierstück 10, ein zweites Reduzierstück 11, das Ventil 7 und ein Druckschlauch-Anschlußstück 12 umfaßt, wobei diese Teile miteinander verschraubt und durch Dichtungsbänder abgedichtet sind. An dem Druckschlauch-Anschlußstück 12 ist der Druckschlauch befestigt.

In der Druckleitung zwischen der CO₂-Gasflasche und dem T-Stück 6 ist ein Rückschlagventil 13 vorgesehen, um zu verhindern, daß Wasser in den Druckminderer 4 der Druckgasflasche eindringt, wenn der Gasdruck abfällt.

Bei Inbetriebnahme der Vorrichtung wird CO₂-Gas aus der Druckgasflasche 1 über das Auslaßventil 2 und den Druckminderer 4 in den Druckschlauch 5 eingespeist. Durch diesen strömt das CO₂-Gas über das Ventil 7 in das T-Stück 6. Mit dem Ventil 7 wird die Zufuhr an CO₂-Gas in das T-Stück auf einfache Weise auf- und zugesperrt. In dem T-Stück 6 wird das zugeführte CO₂-Gas mit dem zulaufenden Leitungswasser vermischt, so daß sich das CO₂-Gas im Wasser löst und dessen pH-Wert infolge von Kohlensäurebildung auf ca. 5,5 bis 6,0 abgesenkt wird. Das kohlensäurehaltige Wasser verläßt das T-Stück 6 und wird der Wasserentnahmestelle zugeführt.

Somit wird eine Vorrichtung zum Einspeisen von Kohlendioxid in Wasser geschaffen, die die einfache und wirkungsvolle Vermischung von CO₂-Gas mit Leitungswasser ermöglicht, wodurch infolge von Kohlensäurebildung das Leitungswasser einen Wert im Bereich von 5,5 bis 6,0 erreicht. Das so zubereitete kohlensäurehaltige Leitungswasser besitzt, wie an sich bekannt, zahlreiche pflegende und gesundheitsfördernde Wirkungen auf Haut und Haare.

## Patentansprüche

1. Vorrichtung zur Einspeisung von CO₂ in Leitungswasser mit einer CO₂-Quelle, die gasförmiges Kohlendioxid unter Druck bereitstellt und aus der das CO₂-Gas dem über eine Wasserleitung zugeführten Leitungswasser vor einer Wasserabgabestelle zugemischt wird, **dadurch gekennzeichnet, daß**
die CO₂-Quelle eine handelsübliche CO₂-Gasflasche (1) ist, und daß
die CO₂-Gasflasche (1) über eine Druckleitung (5) mit einem Anschluß eines T-Stücks (6) verbunden ist, dessen andere Anschlüsse in der Wasserleitung liegen.

2. Vorrichtung nach Anspruch 1, **dadurch gekennzeichnet, daß** in der Druckleitung (5) ein zusätzliches Absperrorgan, insbesondere ein Kugelhahnventil (7), vorgesehen ist.

3. Vorrichtung nach Anspruch 2, **dadurch gekennzeichnet, daß** das Kugelhahnventil mit dem T-Stück (6) integriert ist.

4. Vorrichtung nach einem der Ansprüche 2 oder 3, **dadurch gekennzeichnet, daß** das T-Stück (6) an einem Anschluß ein Gewinde (8) zum Anschrauben an einen Wasserhahn, an einem weiteren Anschluß ein Gewinde (9) zum Anschrauben an einen Duschschlauch und an einem weiteren Anschluß eine Verbindung zum Anschluß an das Ventil (7) aufweist.

5. Vorrichtung nach Anspruch 1, **gekennzeichnet durch** ein Rückschlagventil (13) in der Druckleitung (5) zwischen der CO₂-Gasflasche (1) und dem T-Stück (6).
